# EUROPEAN PATENT SPECIFICATION

(11) **EP 1 658 074 B1**
(45) Date of publication and mention of the grant of the patent: **07.11.2012**
(21) Application number: 04779849.1
(22) Date of filing: 30.07.2004
(51) Int. Cl.: A61K 31/47, A61K 31/17, A61K 31/19, A61P 7/00, A61P 7/06, A61P 33/06, A61P 43/00

(54) **INHIBITORS OF 2-OXOGLUTARATE DIOXYGENASE AS GAMMA GLOBIN INDUCERS**
2-OXOGLUTARAT-OXYGENASE-HEMMER ALS INDUKTOR DES GAMMAGLOBINS
INHIBITEURS DE 2-OXOGLUTARATE DIOXYGENASE EN TANT QU'INDUCTEURS DE GAMMA GLOBINE

(30) Priority: 01.08.2003 US 492045 P
(43) Date of publication of application: 24.05.2006
(73) Proprietor: Fibrogen, Inc., San Francisco, CA 94158 (US); ISIS INNOVATION LIMITED, Summertown, Oxford OX2 7SG (GB)
(72) Inventor: KLAUS, Stephen, J., San Francisco, CA 94109 (US); RATCLIFFE, Peter, J., Kidlington, Oxon OX5 2BA (GB)
(74) Representative: Goodfellow, Hugh Robin
(86) International application number: PCT/US2004/024916
(87) International publication number: WO 2005/011696

(56) References cited:
- WO-A-97/12855
- WO-A-03/053997
- WO-A-2004/004697
- WO-A2-03/049686
- US-A- 5 025 029
- US-A- 5 439 939
- US-A- 5 569 675
- PERRINE S P ET AL: "SODIUM BUTYRATE ENHANCES FETAL GLOBIN GENE EXPRESSION IN ERYTHROID PROGENITORS OF PATIENTS WITH HB SS AND BETA THALASSEMIA" BLOOD, W.B. SAUNDERS, PHILADELPHIA, VA, US, vol. 74, no. 1, 1 July 1989 (1989-07-01), pages 454-459, XP000576149 ISSN: 0006-4971 cited in the application
- DOVER, G.J.; BRUSILOW, S.; CHARACHE, S.: "Induction of Fetal Hemoglobin Production in Subjects With Sickel Cell Anemia by Oral Sodium Phenylbutyrate" BLOOD, vol. 84, no. 1, 1 July 1994 (1994-07-01), pages 339-343, XP002308048 cited in the application
- RODGERS G P, RACHMILEWITZ E A: "NOVEL TREATMENT OPTIONS IN THE SEVERE BETA-GLOBIN DISORDERS" BRITISH JOURNAL OF HAEMATOLOGY, OXFORD, GB, vol. 91, no. 2, 1995, pages 263-268, XP008015323 ISSN: 0007-1048

## Description

### FIELD OF THE INVENTION

The present invention relates to methods and compounds for inducing expression of genes encoding endogenous globin protein. In particular, the invention relates to methods and compounds for enhancing expression of γ-globin.

### BACKGROUND OF THE INVENTION

Hemoglobin, which transports oxygen to tissues of the body, is a tetramer composed of two pairs of polypeptides. The subunit composition and structural and functional character of hemoglobin change during development due to varying oxygen availability and demand. In the embryo, hemoglobin is composed of two ε chains and two ζ chains (ε₂ζ₂). Hemoglobin gene transcription undergoes a switching phenomenon during development, wherein embryonic hemoglobin is replaced by fetal hemoglobin (HbF), which is composed of two α chains and two γ chains (α₂γ₂). Gene switching occurs again in the final weeks before birth, wherein HbF is replaced with adult hemoglobin (HbA), which is composed of two α chains and two β chains (α₂β₂). Thus, in the red cells of normal adults, HbA constitutes about 97% of the total hemoglobin. The remaining 3% is primarily hemoglobin A₂ (α₂δ₂), with HbF (α₂γ₂) accounting for less than 1% of total hemoglobin in normal adult red cells.

The most clinically relevant disorders associated with abnormal hemoglobin are the sickle cell syndromes, wherein a mutation in the β-globin gene generates hemoglobin S (HbS). Upon deoxygenation, HbS forms polymeric fibers, causing red blood cells containing HbS to change from a biconcave disk to an elongated crescent "sickle" shape. The rigid sickle cells form obstructions in blood vessels that result in local tissue hypoxia, further deoxygenation, and further sickling. The result of this cycle is enlargement of the obstruction and increased area of infarction.

Sickling is reduced by the presence of non-S hemoglobin. HbS heterozygotes having one normal β globin gene have fewer clinical problems than HbS homozygotes, which have recurring episodes of pain, chronic hemolytic anemia, and severe infections, usually beginning in early childhood. Sickle β thalassemia occurs when an individual inherits both a sickle β-globin gene and a second defective β-globin gene. If the second β-globin gene produces no β-globin (a truncation mutation; β⁰ thalassemia), the condition is similar to homozygous HbS. In contrast, if the second β-globin gene produces some β-globin protein (e.g., impaired splicing mutation; β⁺ thalassemia), the condition may be less severe with fewer crises, reduced anemia, and less organ damage. Sickle cell disease (SCD) can also lead to gallstones, leg ulcers, bone damage, eye damage, kidney damage, blood sequestration in the spleen and/or liver, pulmonary embolism, and stroke.

Pathophysiological symptoms of SCD are significantly decreased during developmental periods and in various situations where HbF levels are elevated. (See, e.g., Pembrey et al. (1978) Br J Haematol 40:415; Miller et al. (1986) Blood 67:1404; Wood and Weatherall (1983) Biochem J 215:1-10.) While the developmental switch from γ to β globin gene expression is strictly controlled, external factors can influence γ globin gene expression. For example, butyric acid and certain derivatives thereof can delay the fetal to adult hemoglobin switch *in vivo* and increase γ globin gene expression *in vitro* and *in vivo.* (Partington et al. (1984) EMBO J 3:2787-2792; Perrine et al. (1987) Biochem Biophys Res Comm 148:694-700; Perrine et al. (1988) Proc Natl Acad Sci USA 85:8540-8542; Perrine et al. (1989) Blood 74:454-459; Perrine et al. (1993) N Eng J Med 328:81-86; Fibach et al. (1993) Blood 82:2203-2209; U.S. Pat. No. 4,822,821; U.S. Pat. No. 5,025,029; International Publication WO 93/18761.) Additionally, hydroxyurea stimulates globin expression, but the effect is not specific to γ globin. (See, e.g., Letvin et al. (1984) N Engl J Med 310:869-873; Charache et al. (1987) Blood 69:109-16.) Expression from the γ-globin genes has also been successfully manipulated *in vivo* and *in vitro* using agents such as cytosine arabinoside (AraC) (Constantoulakis et al. (1989) Blood 74:1963-71) and 5-azacytidine (AZA) (Ley et al. (1982) N Engl J Med 307:1469-1475).

Recently, a number of aliphatic carboxylic acids, e.g., propionate and pentanoic acid, were shown to specifically increase γ globin, however the positive effects produced by these compounds could be maintained only for very short periods of time. (Safaya et al. (1994) Blood 84:3929-3935; Stamatoyannopoulos et al. (1994) Blood 84:3198-3204.) Other methodologies to increase γ globin expression have focused on recruitment and reprogramming of erythroid progenitor cells to express HbF. Agents tested *in vivo* or *in vitro* include hematopoietic growth factors such as erythropoietin (EPO) (Al-Khatti et al. (1988) Trans Assoc Am Physicians 101:54; Rodgers et al. (1993) N Engl J Med 328:73-80), granulocyte/macrophage-colony stimulating factor (GM-CSF) (Gabbianelli et al. (1989) Blood 74:2657), and interleukin-3 (IL3) (Migliaccio et al. (1990) Blood 76:1150). Each of these factors was found to increase fetal globin synthesis in tissue culture cells. Recent studies have also shown that steel factor, a product of the mouse steel locus, is capable of influencing fetal globin synthesis in erythroid progenitors. (Miller et al. (1992) Blood 79:1861-1868.)

WO 03/053997 discloses methods for treating erythropoietin-associated conditions by increasing endogenous erythropoietin *in vitro* and *in vivo,* as well as methods for treating, pretreating or preconditioning, or preventing erythropoietin-associated condition. Compounds for use in these methods are provided, as are methods of identifying such compounds.

All of the pharmacological therapies currently in use or under investigation exhibit limitations with regard to SCD patients, due to a large percentage of non-responders combined with dose-limiting toxicities and/or pharmacokinetic limitations. For example, 5-azacytidine is a cytostatic and cytotoxic chemotherapeutic agent that inhibits hematopoiesis and displays dose-limiting toxicities with chronic use in humans. Butyrate analogs, on the other hand, display poor pharmacokinetic properties, requiring continuous infusion or ingestion of 40-50 pills per day, and can be associated with, e.g., neurologic toxicity. (See, e.g., Blau et al. (1993) Blood 81:529-537.) Accordingly, additional compounds capable of stimulating the expression of γ globin, and methods for identifying such compounds, are still needed.

Due to limitations and lack of selectivity in current methods for treating hemoglobinopathies, there remains a need for more effective and selective methods for increasing fetal hemoglobin levels in a subject. In particular, there is a need for methods that increase expression of endogenous γ globin and for methods that increase fetal hemoglobin levels. The present invention provides agents that increase fetal hemoglobin by inducing expression of γ-globin in a subject. The agents can be used to selectively and specifically induce fetal hemoglobin in a patient, e.g., to treat a hemoglobinopathy such as β thalassemia or sickle cell anemia.

### SUMMARY OF THE INVENTION

The invention provides an *ex vivo* method for increasing endogenous gamma globin (γ-globin) in a subject cell, the method comprising administering to the cell *ex vivo* an agent which increases the stability or activity of an alpha subunit of hypoxia inducible factor (HIFα). Also provided is an agent which increases the stability or activity of an alpha subunit of hypoxia inducible factor (HIFα) for use in treating or pretreating a subject having a disorder selected from the group consisting of β-thalassemias and sickle cell syndromes by increasing the level of fetal hemoglobin.

The agent may inhibit hydroxylation of HIFα. The HIFα may be any HIFα e.g., a HIFα selected form the group consisting of HIF-1α, HIF-2α, HIF-3α, and any fragment thereof. In some embodiments, the HIFα is endogenous to the subject. In other embodiments, the HIFα may be introduced into the subject e.g., by inserting an expression construct containing a gene encoding the HIFα.

In another embodiment, the agent increases expression of the gene encoding γ-globin by inhibiting 2-oxoglutarate dioxygenase enzyme activity. The 2-oxoglutarate dioxygenase may be any enzyme that requires Fe²⁺, 2-oxoglutarate, and oxygen for enzymatic activity, e.g., modification of a substrate by hydroxylation. Such enzymes include, but are not limited to, procollagen lysyl hydroxylase, procollagen prolyl 3-hydroxylase, procollagen prolyl 4-hydroxylase α(I), α(II), and α(III); thymine 7-hydroxylase, aspartyl (asparaginyl) β-hydroxylase; peroxisomal phytanoyl-CoA a-hydroxylase; ε-N-trimethyllysine hydroxylase and γ-butyrobetaine hydroxylase; EGLN1, EGLN2, and EGLN3; AlkB, PHD4; and factor inhibiting HIF (FIH). In particular embodiments, the 2-oxoglutarate dioxygenase enzyme is selected from the group consisting of EGLN1, EGLN2, EGLN3, PHD4, FIH-1, and any subunit or fragment thereof.

In another embodiment, the agent increases expression of the gene encoding γ-globin by inhibiting HIF hydroxylase enzyme activity. In particular embodiments, the HIF hydroxylase enzyme is selected from the group consisting of EGLN1, EGLN2, EGLN3, FIH-1, and any subunit or fragment thereof.

In one aspect, the invention provides an agent for increasing fetal hemoglobin level in a subject. In one embodiment, agent to be administred to the subject increases expression of the gene encoding γ-globin, thereby increasing fetal hemoglobin level in the subject. The increase in fetal hemoglobin may provide various benefits to the subject. In one embodiment, the agent may be used to treat or pretreat a subject having or at risk for having a disorder associated with abnormal hemoglobin. In various aspects, the abnormal hemoglobin may comprise an alteration in the level, structural integrity, or activity of, e.g., adult β-globin. Such disorders include, but are not limited to, β thalassemias, e.g., β°-and β⁺-thalassemia, and sickle cell syndromes, e.g., sickle trait, sickle β thalassemia, and sickle cell anemia. In another embodiment, the agent may be used to treat or pretreat a subject infected with or at risk for being infected with a species of Plasmodium, e.g., *Plasmodium falciparum.*

In another aspect, the invention provides ex vivo methods for increasing the proportion of fetal hemoglobin relative to non-fetal hemoglobin produced by a cell or population of cells. In one embodiment, the method comprises administering to the cell or population of cells an agent that increases expression of the gene encoding γ-globin.

In one embodiment, the agent may be administered in a pharmaceutical composition wherein the agent is the only therapeutic agent. In other embodiments, the agent may be administered in combination with at least one other therapeutic agent. In one aspect, the additional therapeutic agent may be selected from the group consisting of hydroxyurea, and 5-azacytidine.

In the aspects and embodiments described above, the agent is administered to a subject *in vivo,* e.g., to an animal, particularly to a primate, and more particularly to a human. Alternatively, the agent is administered to a subject *ex vivo,* e.g., to a cell. The cell may be derived, e.g., from bone marrow, or the cell may be selected from the group consisting of, e.g., hematopoietic stem cells and blast-forming unit erythroid (BFU-E) cells. In one embodiment, the agent increases endogenous γ-globin in a population of the cells; and the cells are transfused into a subject, e.g., wherein the subject has a disorder associated with abnormal hemoglobin or is infected with a species of Plasmodium. In various aspects, the population of cells may be selected from the group consisting of hematopoietic stem cells, blast-forming unit erythroid (BFU-E) cells, and bone marrow cells.

In another aspect, the invention provides an agent that increases expression of the gene encoding γ-globin, e.g., for use in increasing fetal hemoglobin level in a subject. In one aspect, the agent is used to treat a disorder associated with abnormal hemoglobin in a subject, e.g., wherein the subject has an alteration in the level, structural integrity, or activity of adult β-globin. Such disorders may include, but are not limited to, β thalassemias, e.g., β⁰- and β⁺-thalassemia, and sickle cell syndromes, e.g., sickle trait, sickle β thalassemia, and sickle cell anemia. In another aspect, the agent may be used to treat or pretreat a subject infected with or at risk for being infected with a species of Plasmodium, e.g., *Plasmodium falciparum*. In another embodiment, the agent may additionally comprise at least one additional therapeutic agent, e.g., a therapeutic agent selected from the group consisting of hydroxyurea, and 5-azacytidine.

### BRIEF DESCRIPTION OF THE DRAWINGS

Figures 1A and 1B show dose-dependent increase in γ-globin expression and resulting fetal hemoglobin accumulation, respectively, in K562 cells treated with various concentrations of HIF-PH inhibitor (HPI) in the presence and absence of added hydroxyurea (HU).

Figure 2 shows induction of HbF in K562 cells by various HPIs. Data are single cultures, with 2 independent cultures of HPI-1 at 20 µM. Induction of HbF by hydroxyurea (HU) is shown for comparison.

Figures 3A and 3B show induction of HbF and increase in HbF-producing cells (F-cells). Figure 3A shows induction of fetal hemoglobin in CD34⁺ human bone marrow progenitor cells by HU and butyrate using HbF-specific antibodies, but no response using an isotype control. Figure 3B shows HPIs stimulate an increase in the percentage of F-cells in cultures of CD34⁺ bone marrow progenitor cells. Data are single cultures, reported as percentage of vehicle control.

Figures 4A and 4B show HPIs specifically induce HbF (Figure 4A) and not total hemoglobin (Figure 4B). Data are from single cultures.

Figures 5A and 5B show HPIs increase HbF as measured by percent F-cells and level of fetal hemoglobin, respectively. Figure 5C shows HbF formed is essentially α₂γ₂, and the effects are additive with those of HU.

Figure 6 shows induction of HbF by various HPIs in CD34⁺ progenitor cell cultures. Data are from single cultures.

### DESCRIPTION OF THE INVENTION

Before the present compositions and methods are described, it is to be understood that the invention is not limited to the particular methodologies, protocols, cell lines, assays, and reagents described, as these may vary. It is also to be understood that the terminology used herein is intended to describe particular embodiments of the present invention, and is in no way intended to limit the scope of the present invention as set forth in the appended claims.

It must be noted that as used herein and in the appended claims, the singular forms "a," "an," and "the" include plural references unless context clearly dictates otherwise. Thus, for example, a reference to "a fragment" includes a plurality of such fragments; a reference to an "antibody" is a reference to one or more antibodies and to equivalents thereof known to those skilled in the art, and so forth.

Unless defined otherwise, all technical and scientific terms used herein have the same meanings as commonly understood by one of ordinary skill in the art to which this invention belongs. Although any methods and materials similar or equivalent to those described herein can be used in the practice or testing of the present invention, the preferred methods, devices, and materials are now described. All publications cited herein are for the purpose of describing and disclosing the methodologies, reagents, and tools reported in the publications that might be used in connection with the invention.

The practice of the present invention will employ, unless otherwise indicated, conventional methods of chemistry, biochemistry, molecular biology, cell biology, genetics, immunology and pharmacology, within the skill of the art. Such techniques are explained fully in the literature. See, e.g., Gennaro, A.R, ed. (1990) Remington's Pharmaceutical Sciences, 18th ed., Mack Publishing Co.; Hardman, J.G., Limbird, L.E., and Gilman, A.G., eds. (2001) The Pharmacological Basis of Therapeutics, 10th ed., McGraw-Hill Co.; Colowick, S. et al., eds., Methods In Enzymology, Academic Press, Inc.; Weir, D.M., and Blackwell, C.C., eds. (1986) Handbook of Experimental Immunology, Vols. I-IV, Blackwell Scientific Publications; Maniatis, T. et al., eds. (1989) Molecular Cloning: A Laboratory Manual, 2nd edition, Vols. I-IITI, Cold Spring Harbor Laboratory Press; Ausubel, F.M. et al., eds. (1999) Short Protocols in Molecular Biology, 4th edition, John Wiley & Sons; Ream et al., eds. (1998) Molecular Biology Techniques: An Intensive Laboratory Course, Academic Press; Newton, C.R., and Graham, A., eds. (1997) PCR (Introduction to Biotechniques Series), 2nd ed., Springer Verlag.

### INVENTION

The present invention provides agents for increasing endogenous γ-globin production. The present methods can be practiced in any subject, e.g., in a cell, tissue, organ, or animal, and can be applied *ex vivo* or *in vivo*. In one aspect, the agents induce γ-globin in cells or tissue cultured *ex vivo*. In certain embodiments, the cells are derived from bone marrow and are hematopoietic stem cells, or early erythroid progenitor cells such as blast-forming unit erythroid (BFU-E) cells. In another aspect, the agents or cells are used on an animal, wherein the animal is a primate, preferably a human, and the agents or cells are used to induce γ-globin expression in endogenous cell populations of the animal.

The invention specifically provides for expresssion of fetal hemoglobin by inducing expression of γ-globin in a subject. Further, the agents or cells can be used to produce fetal hemoglobin in a patient, e.g., to treat a disorder associated with abnormal hemoglobin production, i.e., hemoglobinopathies. Such disorders include β thalassemias, including β⁰-and β⁺-thalassemia, and various sickle cell syndromes, including sickle trait and sickle cell anemia.

The invention further provides methods and agents to increase the level or proportion of fetal hemoglobin (HbF; α₂γ₂) relative to non-fetal hemoglobin, e.g., adult hemoglobin (HbA; α₂β₂ and/or α₂δ₂) produced by a cell or population of cells. The cell or population of cells may be cultured *ex vivo* or be endogenous to and/or resident in an animal such as a primate, preferably a human.

### ME7HODS

The art has demonstrated that hypoxia can increase fetal hemoglobin production in cultured cells and animals. (See, e.g., DeSimone et al. (1978) Proc Natl Acad Sci USA 75(6):2937-2940; DeSimone et al. (1982) Blood 60(2):519-523; and Weinberg et al. (1995) Hemoglobin 19:263-275.) Further, hypoxia has been associated with increased fetal hemoglobin in humans, and increased survival of sickle cell patients. (See, e.g., Bard et al. (1994) J Pediatrics 124:941-943; and Addae et al. (1990) South Med J 83(4):487.) As the form of globin gene expressed at progressive stages of development is highly dependent on oxygen availability and demand, it would be evolutionarily advantageous to place control over the genetic switch of globin gene expression under an oxygen-sensitive mechanism.

The present invention is based on the discovery that regulation of 2-oxoglutarate dioxygenase enzyme activity affects globin gene expression. The 2-oxoglutarate dioxygenase enzymes require 2-oxoglutarate, an intermediate in the citric acid cycle, and oxygen to modify protein substrates. A well characterized member of the 2-oxoglutarate dioxygenase family is procollagen prolyl 4-hydroxylase, which is responsible for post-translational modification of collagen to facilitate collagen trimer formation. More recently described members of the family include the hypoxia inducible factor prolyl hydroxylases (HIF PHs), a family of enzymes that modify the alpha subunit of hypoxia inducible factor (HIFα) and target it for degradation. At low oxygen concentration, HIF PH activity is reduced and HIFα is not modified or degraded. HIFα thus accumulates within the cell, combines with HDFβ, and forms the HIF transcription factor, which activates expression of a repertoire of genes responsible for metabolic shift to promote cell survival and increase vascularization and oxygen-carrying capacity.

It has been shown that stabilization of HIα, e.g., by inhibiting the activity of HIF PH, leads to endogenous erythropoietin production and subsequent increase in hematocrit (See International Publication No. WO 03/053997). The present invention demonstrates that administering compounds that inhibit the activity of 2-oxoglutarate dioxygenase, e.g., HIF PH, leads to increased γ-globin expression and subsequent fetal hemoglobin production in erythroid cells. More particularly, compounds that stabilize HIFα are effective at increasing γ-globin expression, and administration of such compounds to erythroid cells or precursors thereof leads to increased fetal hemoglobin production and increased percentage of HbF-producing cells (F-cells) in the erythrocyte population.

### COMPOUNDS

Several inhibitors of 2-oxoglutarate dioxygenases have been described in general, and inhibitors of various 2-oxoglutarate dioxygenase family members including, e.g., HIF-PH, have been described specifically. (See, Majamaa et al. (1984) Eur J Biochem 138:239-245; Majamaa et al. (1985) Biochem J 229:127-133; Kivirikko and Myllyharju (1998) Matrix Biol 16:357-368; Bickel et al. (1998) Hepatology 28:404-411; Friedman et al. (2000) Proc Natl Acad Sci USA 97:4736-4741; Franklin et al. (2001) Biochem J 353:333-338; Franklin (1991) Biochem Soc Trans 19:812-815; Welford et al. (2003) J Biol Chem 278:10157-10161; and International Publication No. WO 03/049686 .) As used herein, "2-oxoglutarate dioxygenase" encompasses any protein or active fragment thereof that requires Fe²⁺, 2-oxoglutarate, and oxygen for enzymatic activity, e.g., modification of a substrate by hydroxylation. (See, e.g., Majamaa et al. (1985) Biochem J 229:127-133; Myllyharju and Kivirikko (1997) EMBO J 16:1173-1180; Thornburg et al. (1993) Biochemistry 32:14023-14033; and Jia et al. (1994) Proc Natl Acad Sci USA 91:7227-7231.) Such enzymes include, but are not limited to, procollagen lysyl hydroxylase, procollagen prolyl 3-hydroxylase, procollagen prolyl 4-hydroxylase α(I), α(II), and α(III); thymine 7-hydroxylase, aspartyl (asparaginyl) β-hydroxylase; peroxisomal phytanoyl-CoA α-hydroxylase (GenBank Accession No. AAB81834); ε-N-trimethyllysine hydroxylase (e.g., GenBank Accession No. AAL01871) and γ-butyrobetaine hydroxylase (e.g., GenBank Accession No. XP_053891); hypoxia inducible factor prolyl hydroxylase (HIF PH) and factor inhibiting HIF (FIH; e.g., GenBank Accession No. AAL27308; Mahon et al. (2001) Genes Dev 15:2675-2686; Lando et al. (2002) Science 295:858-861; and Lando et al. (2002) Genes Dev 16:1466-1471. Also, see, Elkins et al. (2002) J Biol Chem C200644200). Additionally, 2-oxoglutarate dioxygenase enzymes include AlkB, an enzyme that catalyses demethylation of substrates, e.g., DNA. (See, e.g., Duncan (2002) Proc Natl Acad Sci USA 99:16660-16665.)

Of particular interest are compounds that inhibit one or more HIF hydroxylase. The term "HIF hydroxylase" refers to any enzyme that modifies HIF by hydroxylation of one or more amino acid residues. HIF hydroxylases include Factor Inhibiting HIF (FIH)-1, which modifies at least one asparagine residue found within HIFα (GenBank Accession No. AAL27308; Mahon et al. (2001) Genes Dev 15:2675-2686; Lando et al. (2002) Science 295:858-861; and Lando et al. (2002) Genes Dev 16:1466-1471. Also, see, Elkins et al. (2002) J Biol Chem C200644200.) HIF hydroxylases also include HIF prolyl hydroxylases, which modify proline residues found within HIFα.

The terms "HIF prolyl hydroxylase" and "HIF PH," as used herein, refer to any enzyme that is capable of hydroxylating a proline residue on or associated with an alpha subunit of HIF. In particular embodiments, the proline residue is found within the motif LXXLAP, e.g., as occurs in the human HIF-1α native sequence at L₃₉₇TLLAP and L₅₅₉EMLAP. HIF PH encompasses members of the EGL-9 (EGLN) gene family described by Taylor (2001) Gene 275:125-132; and characterized by Aravind and Koonin (2001) Genome Biol 2:RESEARCH0007; Epstein et al. (2001) Cell 107:43-54; and Bruick and McKnight (2001) Science 294:1337-1340. Examples of HIF PH enzymes include human SM-20 (EGLN1) (GenBank Accession No. AAG33965; Dupuy et al. (2000) Genomics 69:348-54), EGLN2 isoform 1 (GenBank Accession No. CAC42510; Taylor, *supra*), EGLN2 isoform 2 (GenBank Accession No. NP_060025), and EGLN3 (GenBank Accession No. CAC42511; Taylor, *supra*); mouse EGLN1 (GenBank Accession No. CAC42515), EGLN2 (GenBank Accession No. CAC42511), and EGLN3 (SM-20) (GenBank Accession No. CAC42517); and rat SM-20 (GenBank Accession No. AAA19321). Additionally, HIF PH may include *Caenorhabditis elegans* EGL-9 (GenBank Accession No. AAD56365) and *Drosophila melanogaster* CG1114 gene product (GenBank Accession No. AAF52050). HIF PH also includes any active fragment of the foregoing full-length proteins.

The term "HIF PH inhibitor" or "HPI," as used herein, refers to any compound that reduces or otherwise modulates the activity of a HIF PH, as defined herein. An HPI may additionally show inhibitory activity toward one or more other 2-oxoglutarate dioxygenase enzymes, e.g. FIH, procollagen P4H, etc. Specific HPIs that can be used in the invention include, for example, iron chelators, 2-oxoglutarate mimetics, and modified amino acid, e.g., proline, analogs.

In particular embodiments, the present invention provides for use of structural mimetics of 2-oxoglutarate. Such compounds may inhibit the target 2-oxoglutarate dioxygenase enzyme family member competitively with respect to 2-oxoglutarate and noncompetitively with respect to iron. (Majamaa et al. (1984) Eur J Biochem 138:239-245; Majamaa et al. (1985) Biochem J 229:127-133.) HPIs specifically contemplated for use in the present methods are described, e.g., in Majamaa et al., *supra*; Kivirikko and Myllyharju (1998) Matrix Biol 16:357-368; Bickel et al. (1998) Hepatology 28:404-411; Friedman et al. (2000) Proc Natl Acad Sci USA 97:4736-4741; Franklin (1991) Biochem Soc Trans 19:812-815; Franklin et al. (2001) Biochem J 353:333-338; and International Publication No. WO 03/049686. The following exemplary HPIs are used in the present examples to demonstrate the methods of the invention described herein: N-((1-chloro-4-hydroxy-isoquinoline-3-carbonyl)-amino)-acetic acid (HPI-1), [(7-Bromo-4-hydroxy-isoquinoline-3-carbonyo-amino]-acetic acid (HPI-2), [(1-Chloro-4-hydroxy-7-methoxy-isoquinoline-3-carbonyl)-amino]-acetic acid (HPI-3), [(7-Chloro-3-hydroxy-quinoline-2-carbonyl)-amino]-acetic acid (HPI-4), [(3-Hydroxy-6-isopropoxy-quinoline-2-carbonyl)-amino]-acetic acid (HPI-5), and [(4-Hydroxy-7-phenylsulfanyl-isoquinoline-3-carbonyl)-amino]-acetic acid (HPI-6).

Additional compounds for use in the present invention may be identified by their ability to inhibit hydroxylation of a HIFα subunit, in particular, a proline residue of HIFα As used herein, the term "HIFα" refers to the alpha subunit of hypoxia inducible factor protein. HIFα may be any human or other mammalian protein, or fragment thereof, including, but not limited to, human HIF-1α (Genbank Accession No. Q16665), HIF-2α (Genbank Accession No. AAB41495), and HIF-3α (Genbank Accession No. AAD22668); murine HIF-1α (Genbank Accession No. Q61221), HM-2α(Genbank Accession No. BAA20130 and AAB41496), and HIF-3α (Genbank Accession No. AAC72734); rat HIF-1α (Genbank Accession No. CAA70701), HIF-2α (Genbank Accession No. CAB96612), and HIF-3α (Genbank Accession No. CAB96611); and cow HIF-1α (Genbank Accession No. BAA78675). HIFα may also be any non-mammalian protein or fragment thereof, including Xenopus laevis HIF-1α (Genbank Accession No. CAB96628), Drosophila melanogaster HIF-1α (Genbank Accession No. JC4851), and chicken HIF-1α (Genbank Accession No. BAA34234).

Screening methods for identifying further suitable compounds may utilize full-length HIFα, as described above, or fragments of HIFα. For example, HIFα fragments for use in an assay to identify compounds that inhibit HIF PH may include regions of HIFα defined by human HIF-1α from amino acid 401 to 603 (Huang et al., *supra*), amino acid 531 to 575 (Jiang et al. (1997) J Biol Chem 272:19253-19260), amino acid 556 to 575 (Tanimoto et al., *supra*), amino acid 557 to 571 (Srinivas et al. (1999) Biochem Biophys Res Commun 260:557-561), or amino acid 556 to 575 (Ivan and Kaelin (2001) Science 292:464-468). More generally, peptides for use in the screening method may include any fragment containing at least one occurrence of the motif LXXLAP, e.g., as occurs in the HIF-1α native sequence at L₃₉₇TLLAP and L₅₅₉EMLAP.

In one embodiment of the disclosure, a screening method for identifying a suitable compound comprises combining a HIF PH with at least a fragment of HIFα in the presence of compound, measuring hydroxylation, e.g., proline hydroxylation, of HIFα, and comparing the level of hydroxylation in the presence of compound to the level of hydroxylation when the same assay is carried out in the absence of compound. A difference in hydroxylation when compound is present as compared to when compound is absent is indicative of a compound that modulates HIF PH.

In some embodiments of the disclosure, the screening method is conducted in cell-free system using purified or partially purified components, e.g., HIF PH, HIFα, etc. Detection in cell-flee systems may involve methods of direct peptide analysis, e.g., thin layer chromatography, HPLC, etc., to determine level of hydroxyproline in the HIFα protein or peptide. Alternatively, detection may involve indirect measurement, e.g., measuring formation of a coproduct of the reaction such as carbon dioxide, succinate, etc.

In other embodiments of the disclosure, the screening method is conducted *in vitro* or *ex vivo*, in cells or tissues wherein the HIF PH and/or HIFα are endogenous to the cell or tissue. Detection in cell-based assays may involve, e.g., measuring the level of HIFα in cellular or nuclear fractions obtained from cell lysates. Alternatively, detection, as exemplified herein, may involve measuring expression of γ-globin and/or production of fetal hemoglobin in the cell. In particular embodiments of the disclosure, the cell is select from a K562 human erythroleukemia cell (American Type Culture Collection (ATCC), Manassas VA) or a human CD34⁺ bone marrow progenitor cell (Cambrex Bioscience, Baltimore MD).

### THERAPEUTICS

In one embodiment, agents and/or cells of the present invention are used to treat a disease or disorder in a patient in need. In some aspects, the agents and/or cells are used to treat or pre-treat an individual having or at risk for having a hemoglobinopathy. Such hemoglobinopathies include any disorder associated with an alteration in the amount, structural integrity, or function of adult hemoglobin, specifically adult β globin. Hemoglobinopathies specifically include, but are not limited to, β thalassemia including β°. (major) and β⁺- (minor) thalassemia, and sickle cell disease including sickle cell anemia and sickle β thalassemia. In other aspects, the agents and/or cells of the invention are used to treat or pre-treat an individual infected with or at risk for being infected with a Plasmodium species, e.g., *Plasmodium falciparum*, which is associated with malaria.

In some embodiments, to a patient in need an effective amount of a compound that increases γ globin production in a cell or population of cells is to be administered. In one aspect, the compound inhibits the activity of a 2-oxoglutarate dioxygenase, and in a specific aspect, the compound inhibits the activity of HIF-PH. The compound may be administered alone, in a pharmaceutically acceptable formulation, or in combination with a second therapeutic agent. In some embodiments, the second therapeutic agent additively or synergistically increases γ globin production. Such agents include, but are not limited to, hydroxyurea, butyrate analogs, and 5-azacytidine. (See, e.g., U.S. Pat No. 5,569,675; U.S. Pat No. 5,700,640; U.S. Pat No. 6,231,880; International Publication No. WO 93/18761; and International Publication No. WO 97/12855.) In other embodiments, the second therapeutic agent provides therapeutic benefit to patients in need by a mechanism distinct from γ globin induction, e.g., such agents may include, but are not limited to, Gardos channel inhibitors (see, e.g., U.S. Pat. No. 6,218,122; U.S. Pat No 6,331,564; International Publication No. WO 99/24034; and International Publication No. WO 96/08242), which inhibit dehydration of red blood cells; and/or hematopoietic growth factors such as EPO, GM-CSF, and IL3.

Continuous formation and destruction of irreversibly sickled cells contributes significantly to the severe hemolytic anemia that occurs in patients with sickle cell disease. The anemia of sickle cell can be even more severe if erythropoiesis is suppressed. For example, folic acid and vitamin B₁₂ are required for proper cell division; and deficiency in these nutrients leads to enlarged blood cells (megaloblastic cells), which are destroyed in the marrow, thus causing anemia due to ineffective erythropoiesis. Further, a deficiency in iron, which is necessary for functional heme production, further aggravates the anemia. Therefore, in yet another embodiment, the compound is to be administered with a second agent selected from the group consisting of folic acid, vitamin B₁₂, and an iron supplement, e.g., ferrous gluconate.

In another embodiment, an agent which increases the stability of on alpha subunit of HIFX is used to treat cells *ex vivo* to induce γ-globin production. The cells may be transfused into a patient in need. In a particular embodiment, the cells are bone marrow cells. The bone marrow may be derived from the patient (autologous) or from a matched donor (allogenic). Such methods can be used in conjunction with current blood transfusions, e.g., for treatment of SCD crises.

In another embodiment, a patient in need is to be treated with an HPI to induce γ globin expression and HbF production. The use of an HPI can provide additional benefits in various disorders by mitigating damage related to ischemia-reperfusion damage, as seen in acute and chronic ischemic crises in SCD patients. Specifically, hypoxia inducible factor has been associated with expression of various proteins including, but not limited to, heme oxygenase (HO)-1, inducible nitric oxide synthase (iNOS), superoxide dismutase (SOD), glycolytic enzymes, adrenomedullin, and erythropoietin (EPO). (See, e.g., International Publication No. WO 03/049686.) Several of these proteins provide direct benefit in SCD patients; e.g., heme oxygenase facilitates removal of hemoglobin released from damaged red blood cells, nitric oxide leads to vasodilation which facilitates movement of blood cells through vessels, and EPO enhances erythropoiesis and potentially augments γ globin synthesis.

Additionally, as patients with sickle cell disease can secondarily accrue chronic organ damage, especially of the lungs, kidneys, liver, skeleton, and skin, due to the cumulative effects of recurrent vasoocclusive episodes (pulmonary infarcts with associated congestive heart failure; skeletal infarction, which generally leads to increased bony trabeculation and sclerosis; chronic skin ulcers, especially in distal lower extremities, e.g. ankle ulcers), the agents and/or cells of the invention may provide additional benefits associated with stabilization of HIFα including cytoprotective and angiogenic effects. Such additional benefits can be achieved, as described above, by selective use of an HPI.

In some aspects, the agents and/or cells are for use to treat patients with SCD who do not respond to treatment with other therapeutic agents, e.g., hydroxyurea, etc. In other aspects, the agents and/or cells for use to augment treatment with another therapeutic agent, e.g. hydroxyurea, etc. The use of the current agents and/or cells in conjunction with a second therapeutic may be required because, e.g., the patient may only partially respond to current therapy. The present invention demonstrates an additive or synergistic increase in HbF is achieved when PHIs are used in conjunction with, e.g., hydroxyurea or butyrate. Further, the use of the current agents and/or cells in conjunction with a second therapeutic agent may facilitate reducing the required dosage of the second agent to avoid pharmacokinetic or toxicity associated with the agent. For example, current therapies in hemoglobinopathies are limited by factors including poor pharmacokinetics, e.g., butyrate requires extremely high doses for therapeutic efficacy (Dover et al. (1994) Blood 84(1):339-343); and dose-limiting toxicity associated with, e.g. butyrate, 5-azacytidine, and hydroxyurea. (See, e.g., Blau et al. (1993) Blood 81:529-537; Ley and Nienhuis (1985) Annu Rev Med 36:485-498; DeSimone et al. (2002) Blood 99:3905-3908; and Charache et al. (1995) N Engl J Med 332:1317-1322.)

Therefore, the present invention provides an HPI in combination with a second therapeutic agent such as hydroxyurea for administration to a patient in need. The ratio of HPI to second therapeutic agent can be readily determined by one of skill in the art, and is calculated to facilitate treatment of patients who respond poorly to the second agent when administered alone; to facilitate treatment of patients with a lower dose of second agent, e.g., to reduce associated secondary pharmacological or toxicological effects of the second agent; or to produce a synergistic response that allows intermittent administration of the second agent. In certain embodiments, an HPI is for administration with a second therapeutic agent to increase expression of γ-globin in a patient in need. While HPIs may be additive or synergistic with the second agent for expression of γ-globin and/or production of HbF, additional benefit may also be derived from the combined action of an HPI and second agent, such as hydroxyurea, on increasing bioavailability of nitric oxide (NO). (See, e.g., Jiang et al. (1997) Mol Pharmacol 52(6):1081-1086; Cokic et al. (2003) J Clin Invest 111 (2):231-239.) In addition, HPI can increase heme oxygenase-1 expression, which acts to catabolize free heme and further increase NO bioavailability.

### Medicaments And Routes Of Administration

The compounds, e.g, HPIs, of the present invention can be delivered directly or in pharmaceutical compositions along with suitable carriers or excipients, as is well known in the art. Treatment can comprise administration of an effective amount of a compound of the invention to a subject having or at risk for hemoglobinopathies including β thalassemia major, β thalassemia minor, sickle cell disease, etc. In a preferred embodiment, the subject is a primate, and in a most preferred embodiment, the subject is a human.

An effective amount, e.g., dose, of compound or drug can readily be determined by routine experimentation, as can an effective and convenient route of administration and an appropriate formulation. Various formulations and drug delivery systems are available in the art. (See, e.g., Gennaro, Ed. (2000) Remington's Pharmaceutical Sciences, supra; and Hardman, Limbird, and Gilman, Eds. (2001) The Pharmacological Basis of Therapeutics, supra.)

Suitable routes of administration may, for example, include oral, rectal, topical, nasal, pulmonary, ocular, intestinal, and parenteral administration. Primary routes for parenteral administration include intravenous, intramuscular, and subcutaneous administration. Secondary routes of administration include intraperitoneal, intra-arterial, intra-articular, intracardiac, intracisternal, intradermal, intralesional, intraocular, intrapleural, intrathecal, intrauterine, and intraventricular administration. The indication to be treated, along with the physical, chemical, and biological properties of the drug, dictate the type of formulation and the route of administration to be used, as well as whether local or systemic delivery would be preferred.

Pharmaceutical dosage forms of a compound of the invention may be provided in an instant release, controlled release, sustained release, or target drug-delivery system. Commonly used dosage forms include, for example, solutions and suspensions, (micro-) emulsions, ointments, gels and patches, liposomes, tablets, dragees, soft or hard shell capsules, suppositories, ovules, implants, amorphous or crystalline powders, aerosols, and lyophilized formulations. Depending on route of administration used, special devices may be required for application or administration of the drug, such as, for example, syringes and needles, inhalers, pumps, injection pens, applicators, or special flasks. Pharmaceutical dosage forms are often composed of the drug, an excipient(s), and a container/closure system. One or multiple excipients, also referred to as inactive ingredients, can be added to a compound of the invention to improve or facilitate manufacturing, stability, administration, and safety of the drug, and can provide a means to achieve a desired drug release profile. Therefore, the type of excipient(s) to be added to the drug can depend on various factors, such as, for example, the physical and chemical properties of the drug, the route of administration, and the manufacturing procedure. Pharmaceutically acceptable excipients are available in the art, and include those listed in various pharmacopoeias. (See, e.g., the U.S. Pharmacopeia (USP), Japanese Pharmacopoeia (JP), European Pharmacopoeia (EP), and British pharmacopeia (BP); the U.S. Food and Drug Administration (www.fda.gov) Center for Drug Evaluation and Research (CEDR) publications, e.g., Inactive Ingredient Guide (1996); Ash and Ash, Eds. (2002) Handbook of Pharmaceutical Additives, Synapse Information Resources, Inc., Endicott NY; etc.)

Pharmaceutical dosage forms of a compound of the present invention may be manufactured by any of the methods well-known in the art, such as, for example, by conventional mixing, sieving, dissolving, melting, granulating, dragee-making, tabletting, suspending, extruding, spray-drying, levigating, emulsifying, (nano/micro-) encapsulating, entrapping, or lyophilization processes. As noted above, the compositions of the present invention can include one or more physiologically acceptable inactive ingredients that facilitate processing of active molecules into preparations for pharmaceutical use.

Proper formulation is dependent upon the desired route of administration. For intravenous injection, for example, the composition may be formulated in aqueous solution, if necessary using physiologically compatible buffers, including, for example, phosphate, histidine, or citrate for adjustment of the formulation pH, and a tonicity agent, such as, for example, sodium chloride or dextrose. For transmucosal or nasal administration, semisolid, liquid formulations, or patches may be preferred, possibly containing penetration enhancers. Such penetrants are generally known in the art. For oral administration, the compounds can be formulated in liquid or solid dosage forms and as instant or controlled/sustained release formulations. Suitable dosage forms for oral ingestion by a subject include tablets, pills, dragees, hard and soft shell capsules, liquids, gels, syrups, slurries, suspensions, and emulsions. The compounds may also be formulated in rectal compositions, such as suppositories or retention enemas, e.g., containing conventional suppository bases such as cocoa butter or other glycerides.

Solid oral dosage forms can be obtained using excipients, which may include, fillers, disintegrants, binders (dry and wet), dissolution retardants, lubricants, glidants, antiadherants, cationic exchange resins, wetting agents, antioxidants, preservatives, coloring, and flavoring agents. These excipients can be of synthetic or natural source. Examples of such excipients include cellulose derivatives, citric acid, dicalcium phosphate, gelatine, magnesium carbonate, magnesium/sodium lauryl sulfate, mannitol, polyethylene glycol, polyvinyl pyrrolidone, silicates, silicium dioxide, sodium benzoate, sorbitol, starches, stearic acid or a salt thereof, sugars (i.e. dextrose, sucrose, lactose, etc.), talc, tragacanth mucilage, vegetable oils (hydrogenated), and waxes. Ethanol and water may serve as granulation aides. In certain instances, coating of tablets with, for example, a taste-masking film, a stomach acid resistant film, or a release-retarding film is desirable. Natural and synthetic polymers, in combination with colorants, sugars, and organic solvents or water, are often used to coat tablets, resulting in dragees. When a capsule is preferred over a tablet, the drug powder, suspension, or solution thereof can be delivered in a compatible hard or soft shell capsule.

In one embodiment, the compounds of the present invention can be administered topically, such as through a skin patch, a semi-solid, or a liquid formulation, for example a gel, a (micro-) emulsion, an ointment, a solution, a (nano/micro)-suspension, or a foam. The penetration of the drug into the skin and underlying tissues can be regulated, for example, using penetration enhancers; the appropriate choice and combination of lipophilic, hydrophilic, and amphiphilic excipients, including water, organic solvents, waxes, oils, synthetic and natural polymers, surfactants, emulsifiers; by pH adjustment; and use of complexing agents. Other techniques, such as iontophoresis, may be used to regulate skin penetration of a compound of the invention. Transdermal or topical administration would be preferred, for example, in situations in which local delivery with minimal systemic exposure is desired.

For administration by inhalation, or administration to the nose, the compounds for use according to the present invention are conveniently delivered in the form of a solution, suspension, emulsion, or semisolid aerosol from pressurized packs, or a nebuliser, usually with the use of a propellant, e.g., halogenated carbons dervided from methan and ethan, carbon dioxide, or any other suitable gas. For topical aerosols, hydrocarbons like butane, isobutene, and pentane are useful. In the case of a pressurized aerosol, the appropriate dosage unit may be determined by providing a valve to deliver a metered amount. Capsules and cartridges of, for example, gelatin, for use in an inhaler or insufflator, may be formulated. These typically contain a powder mix of the compound and a suitable powder base such as lactose or starch.

Compositions formulated for parenteral administration by injection are usually sterile and, can be presented in unit dosage forms, e.g., in ampoules, syringes, injection pens, or in multi-dose containers, the latter usually containing a preservative. The compositions may take such forms as suspensions, solutions, or emulsions in oily or aqueous vehicles, and may contain formulatory agents, such as buffers, tonicity agents, viscosity enhancing agents, surfactants, suspending and dispersing agents, antioxidants, biocompatible polymers, chelating agents, and preservatives. Depending on the injection site, the vehicle may contain water, a synthetic or vegetable oil, and/or organic co-solvents. In certain instances, such as with a lyophilized product or a concentrate, the parenteral formulation would be reconstituted or diluted prior to administration. Depot formulations, providing controlled or sustained release of a compound of the invention, may include injectable suspensions of nano/micro particles or nano/micro or non-micronized crystals. Polymers such as poly(lactic acid), poly(glycolic acid), or copolymers thereof, can serve as controlled/sustained release matrices, in addition to others well known in the art. Other depot delivery systems may be presented in form of implants and pumps requiring incision.

Suitable carriers for intravenous injection for the molecules of the invention are well-known in the art and include water-based solutions containing a base, such as, for example, sodium hydroxide, to form an ionized compound, sucrose or sodium chloride as a tonicity agent, for example, the buffer contains phosphate or histidine. Co-solvents, such as, for example, polyethylene glycols, may be added. These water-based systems are effective at dissolving compounds of the invention and produce low toxicity upon systemic administration. The proportions of the components of a solution system may be varied considerably, without destroying solubility and toxicity characteristics. Furthermore, the identity of the components may be varied. For example, low-toxicity surfactants, such as polysorbates or poloxamers, may be used, as can polyethylene glycol or other co-solvents, biocompatible polymers such as polyvinyl pyrrolidone may be added, and other sugars and polyols may substitute for dextrose.

For composition useful for treatment, a therapeutically effective dose can be estimated initially using a variety of techniques well known in the art. Initial doses used in animal studies may be based on effective concentrations established in cell culture assays. Dosage ranges appropriate for human subjects can be determined, for example, using data obtained from animal studies and cell culture assays.

An effective amount or a therapeutically effective amount or dose of an agent, e.g., a compound of the invention, refers to that amount of the agent or compound that results in amelioration of symptoms or a prolongation of survival in a subject. Toxicity and therapeutic efficacy of such molecules can be determined by standard pharmaceutical procedures in cell cultures or experimental animals, e.g., by determining the LD50 (the dose lethal to 50% of the population) and the ED50 (the dose therapeutically effective in 50% of the population). The dose ratio of toxic to therapeutic effects is the therapeutic index, which can be expressed as the ratio LD50/ ED50. Agents that exhibit high therapeutic indices are preferred.

The effective amount or therapeutically effective amount is the amount of the compound or medicament that will elicit the biological or medical response of a tissue, system, animal or human that is being sought by the researcher, veterinarian, medical doctor or other clinician, e.g., increased percentage F-cells and/or HbF in circulation, reduced SCD crises, etc.

Dosages preferably fall within a range of circulating concentrations that includes the ED50 with little or no toxicity. Dosages may vary within this range depending upon the dosage form employed and/or the route of administration utilized. The exact formulation, route of administration, dosage, and dosage interval should be chosen according to methods known in the art, in view of the specifics of a subject's condition.

Dosage amount and interval may be adjusted individually to provide plasma levels of the active moiety that are sufficient to achieve the desired effects, e.g., increase in fetal hemoglobin level and/or percent circulating F-cells relative to total RBCs, etc.; i.e., the minimal effective concentration (MEC). The MEC will vary for each compound but can be estimated from, for example, in vitro data and animal experiments. Dosages necessary to achieve the MEC will depend on individual characteristics and route of administration. In cases of local administration or selective uptake, the effective local concentration of the drug may not be related to plasma concentration.

The amount of agent or composition administered may be dependent on a variety of factors, including the sex, age, and weight of the subject being treated, the severity of the affliction, the manner of administration, and the judgment of the prescribing physician.

The present compositions may, if desired, be presented in a pack or dispenser device containing one or more unit dosage forms containing the active ingredient. Such a pack or device may, for example, comprise metal or plastic foil, such as a blister pack, or glass and rubber stoppers such as in vials. The pack or dispenser device may be accompanied by instructions for administration. Compositions comprising a compound of the invention formulated in a compatible pharmaceutical carrier may also be prepared, placed in an appropriate container, and labeled for treatment of an indicated condition. Suitable conditions indicated on the label may include treatment of hemoglobinopathies.

These and other embodiments of the present invention will readily occur to those of ordinary skill in the art in view of the disclosure herein.

### EXAMPLES

The invention will be further understood by reference to the following examples, which are intended to be purely exemplary of the invention. These examples are provided solely to illustrate the claimed invention. The present invention is not limited in scope by the exemplified embodiments, which are intended as illustrations of single aspects of the invention only. Various modifications of the invention in addition to those described herein will become apparent to those skilled in the art from the foregoing description and accompanying figures.

### Example 1: Test materials.

Compounds of the present invention were synthesized using standard chemical methods known to those of skill in the art. Compounds were analyzed for purity by high pressure liquid chromatography and stored at room temperature protected from light. During formulation for various uses, compounds were micronized in suspension at either 500 rpm for 25 minutes or 750 rpm for 10 min using a PULVERISETTE 7 planetary micro mill (Fritsch GMBH, Germany) to facilitate uniform particle size.

Suspensions of micronized compound for oral gavage were prepared immediately before use. Compound was suspended in aqueous solution containing 0.5% sodium carboxymethylcellulose (CMC; Spectrum Chemical, Gardena CA), 0.1% polysorbate 80 (Mallinckrodt Baker, Inc., Phillipsburg NJ) and stirred constantly using a magnetic stirrer or rotary shaker during dose administration. The concentration of the suspensions was calculated to achieve the intended dose level in a given volume. In alternative procedures, compound was weighed and placed in appropriately sized gelatin capsules for oral administration, wherein control animals received empty capsules of the same size; or compound was dissolved in a 100 mM histidine (Mallinckrodt Baker) solution and provided *ad libitum* in place of water.

For administration by injection, compound was initially mixed with an equimolar amount of sodium hydroxide, in either an aqueous solution of 10% glucose (Spectrum) or 25 mM histidine combined with sodium chloride at isotonicity (Mallinckrodt Baker).

### Example 2: Cell Culture

For studies utilizing the human erythroleukemia K562 cell line (ATCC), cells were grown and cultured in complete medium (RPMI 1640, 10% fetal calf serum (FCS), 100 U/ml penicillin and 0.1 mg/ml streptomycin) in a humidified incubator at 37°C, 95% air, 5% CO₂. HPIs were titrated into duplicate cultures at dosage levels spanning more than 100-fold differences in concentration, and incubated from 24 to 96 hours prior to harvesting and quantitation of F-cells and HbF levels by flow cytometry. Optimal concentrations of hydroxyurea that alone induced HbF were determined empirically, and were used as positive control.

For studies utilizing human CD34⁺ bone marrow progenitors (Cambrex Bioscience), cells were cultured according to established protocols for erythroid differentiation (Fibach et al. (1989) Blood 73:100-103). In the EPO-independent phase of the culture (Phase I), cells were plated at 10⁴ cells per well in 6-well plates and cultured for 1 week in HPGM media (Cambrex) supplemented with a cytokine cocktail containing 50 ng/ml each of stem cell factor (SCF), granulocyte-macrophage colony stimulating factor (GM-CSF), interleukin-6 (IL-6), and IL-3 (R&D Systems, Minneapolis MN). In the EPO-dependent phase (Phase II), cells were cultured in HPGM containing the identical cytokine cocktail as in phase I plus 1. U/ml of recombinant EPO (R&D Systems), and cultured for another 10-14 days depending on the magnitude and duration of EPO-dependent proliferation that occurred. After the first week of phase II, the cell concentration was adjusted so that cell density did not exceed 2.5 x 10⁶ cells/ml.

The cells were tested for appropriate HPI concentration as described for K562 cells. HPIs were administered to CD34⁺ cells using one of the following protocols: HPI or vehicle control was added to cell cultures (1) only during Phase I of the erythropoeitic differentiation protocol, (2) only during Phase II, or (3) during both Phase I and II. Hydroxyurea was added only during Phase II of the culture due to its negative effects on cell proliferation and recovery. Experience to date shows that the CD34⁺ progenitor cells routinely expand from 400-500-fold during the full 3-week culture period, ensuring sufficient cells per sample for immunostaining and flow cytometry.

For flow cytometric analysis of F-cells and HbF levels, cells were analyzed using commercially available reagents that detect HbF and total Hb. Following phase I and II of the culture period, cells were harvested and approximately 1-2 X 10⁵ cells were placed into round-bottom 96-well microtiter plates for each flow cytometry sample. The assay buffer for all washes and the diluent used for antibodies is PBS containing 0.1% BSA. All incubations with antibodies and centrifugation steps were conducted at 4°C and protected from light.

For HbF and Hb-specific immunolabeling, cells were pelleted by centrifugation at 1200 rpm for 2 minutes in a swinging bucket centrifuge (Beckman Coulter, Fullerton CA) with adaptors for microtiter plates, and the supernatant was aspirated. The cells were resuspended and lightly fixed in 100 ml of buffer containing 0.05% glutaraldehyde for 10 minutes, followed by addition of 100 ml of buffer and pelleting by centrifugation exactly as described above. The cells were washed two more times (for a total of 2.5 washes) before resuspension in buffer containing 1% Triton-X 100 for 5 min, to permeabilize the cells and enable penetration of antibodies. Following an additional 2.5 washes as described, cells were resuspended with 100 ml of buffer containing antibodies specific for HbF or the appropriate monoclonal isotype control (Caltag Laboratories, Burlingame CA), or total Hb and the matched polyclonal isotype control (BiosPacific, Emeryville CA). The final concentration of antibodies was between 2.5 to 10 µg/ml.

The antibodies for HbF and isotype-matched control were directly conjugated to fluorochromes, so after antibody incubation the cells were washed as above before fixation in 1% formaldehyde, and stored at 4°C in the dark until analysis. With immunostaining for total Hb (and isotype-matched control), a second step was necessary using FITC-conjugated rabbit anti-goat IgG (Jackson Immunoresearch Laboratories, West Grove PA) at a final concentration of 10 µg/ml. After an additional 2.5 washes, cells were fixed in 1% formaldehyde and stored at 4°C in the dark until analysis. All flow cytometric analysis was conducted on a FACSCAN system (BD Bioscience, San Jose CA).

The mean fluorescence intensity (MFI) of HbF expression within individual samples was determined relative to the MFI obtained from isotype control antibodies. The MFI was corrected for background fluorescence, and then MFI for HPI- or HU-treated cultures was compared to MFI for vehicle control. To determine the percent HbF-positive cells (F-cells) in individual cultures, a fluorescence histogram of cell samples stained with isotype control antibodies was generated, and the filter was gated for the top two percent of the cell distribution. Cells stained with HbF-specific antibodies were then analyzed, and the percentage of cells in the histogram displaying MFI above the gated value established with isotype control were considered to be F-cells. Cell cultures established from individual donors contain varying percentages of F-cells (anywhere from 2.5% to 50% after a 3 week culture *in vitro*) in the absence of HPI or HU treatment. Therefore, the percent increase in F-cells was determined by comparing the percent of F-cells in cultures treated with HPI or HU to percent F-cells in cultures treated with vehicle control.

### Example 3: Animal dosing

Healthy male and/or female baboons, e.g., *Papio cynocephalus*, or rhesus monkeys, e.g., *Macaca mulatta* (approximately 1.5 - 4 years old) are obtained and cared for according to standard protocols in an approved primate center. Experiments are carried out as described previously. (See, e.g., Letvin et al. (1984) N Engl J Med 310:869-873; Constantoulakis et al. (1988) Blood 72:1961-1967; and McDonagh et al. (1992) Exp Hematol 20:1156-1164). Animals are maintained using standard procedures, and water is available to the animals *ad libitum*. During treatment, animals are monitored for changes in body weight and signs of overt toxicity and mortality.

Compounds are generally administered orally by gavage or gelatin capsules. Animals treated by oral gavage receive a 4 ml/kg volume of either 0.5% carboxymethyl cellulose (CMC; Sigma-Aldrich, St. Louis MO) (0 mg/kg/day) or varying doses of an HPI in 0.5% CMC. Animals are anesthetized and blood samples are collected at appropriate intervals during treatment. Hemoglobin levels, reticulocyte counts, white-cell counts, and differential cell counts are determined using standard techniques. Peripheral-blood red cells that contain fetal hemoglobin (F-cells) are counted by direct assessment of acid-elution-resistant erythrocytes on blood smears. Alternatively, the percentage of HbF in red cell lysates is quantitated by any method routinely available in the art, e.g., high-pressure liquid chromatography, flow cytometry, ion exchange chromatography, etc. (See, e.g., Wilson et al. (1983) J Lab Clin Med 102:174; Example 2, *supra;* and Example 9, *infra*.) HPIs are also evaluated for additive or synergistic effects with HU as described in Example 2.

### Example 4: Increased γ-globin expression and induction of HbF in K562 cells

Analysis of γ-globin expression and fetal hemoglobin induction by HPIs *in vitro* was performed using the K562 cell line as described in Example 2. In one experiment, cells were cultured for 3 days in media containing 0, 3, 4.5, 10, 12.5, or 20 µM HPI-6. Fetal hemoglobin induction was evaluated by flow cytometry as described above. Expression of γ-globin mRNA was measured as follows. RNA was isolated from cells using an RNEASY MINI kit (Qiagen, Inc., Valencia CA) according to the manufacturer's instructions. Isolated RNA was treated with DNase I (Ambion, Inc., Austin TX), and then cDNA was prepared from total RNA using 1 µM random hexamer primers, 250 ng total RNA, and OMNISCRIPT reverse transcriptase (Qiagen, Inc., Valencia CA), according to the manufacturer's instructions. Resulting cDNA was diluted 10-fold with water, and relative γ-globin mRNA level was measured by quantitative PCR using SYBR GREEN MASTER PCR mix (Applied Biosystems, Inc., Foster City CA) and human γ-globin-specific primers (Sigma-Aldrich, St. Louis MO), using an ABI PRISM 7000 system (Applied Biosystems), according to manufacturer's instructions. Samples were heated to 95°C for 15 minutes and then cycled through 95°C for 15 seconds, 60°C for 60 seconds for a total of 40 cycles.

Relative 18S ribosomal RNA mRNA level was also measured using SYBR GREEN MASTER PCR mix (Applied Biosystems), human 18s rRNA-specific primers (Sigma-Aldrich), and the ABI PRISM 7000 system (Applied Biosystems) according to manufacturer's instructions. Samples were heated to 95°C for 15 minutes and then cycled through 94°C for 15 seconds, 60°C for 60 seconds, for a total of 40 cycles.

Each PCR run included a standard curve and water blank. In addition, a melt curve was run after completion of each PCR run to assess the specificity of the amplification. γ-globin gene expression was normalized relative to the expression level of 18S ribosomal RNA for each sample.

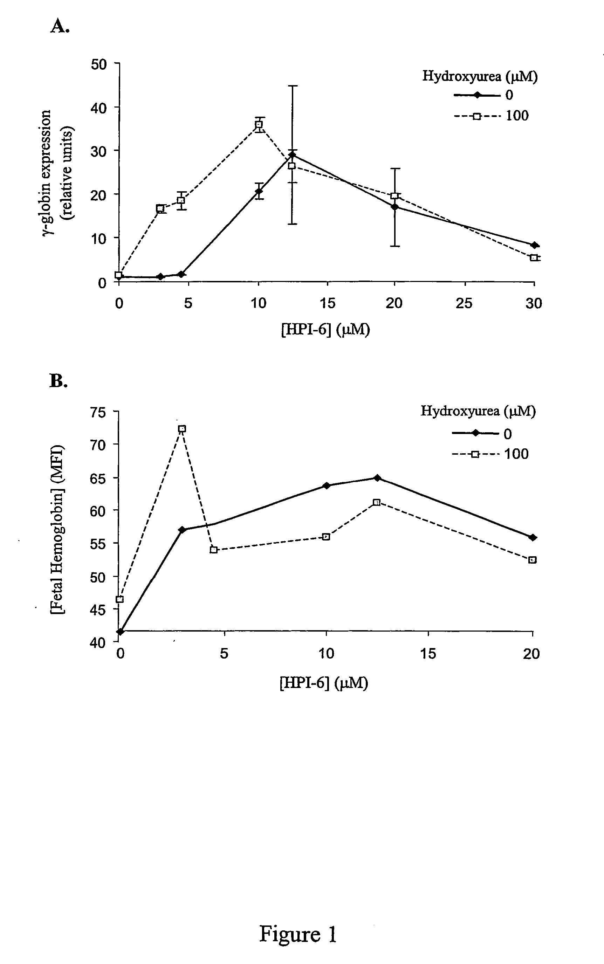

As shown in Figure 1A, expression of the gene encoding γ-globin increased in a dose-dependant manner following treatment with HPI-6. Further, γ-globin expression was augmented, especially at lower PHI concentrations, by further addition of 100 µM hydroxyurea (HU). Similarly, HPIs increased HbF level in a dose-dependent manner, and augmented HbF production induced by HU. (Figure 1B.) Thus, HPIs increase γ-globin expression and HbF level in cells, and provide additive benefit when used in combination with agents such as hydroxyurea.

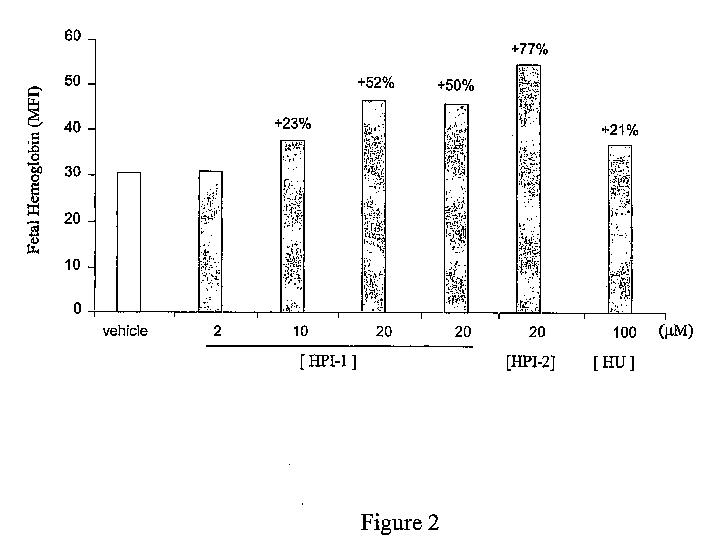

In a separate experiment, cells were cultured for 4 days in media containing 2, 10, or 20 µM HPI-1, 20 µM HPI-2, 100 µM HU, or vehicle control (DMSO). Fetal hemoglobin induction was then evaluated by flow cytometry as described above. As can be seen in Figure 2, 20 µM HPI-1 stimulated a 23-52% increase in HbF synthesis compared to negative control cultures. Similarly, 20 µM PHI-2 stimulated a 77% increase relative to negative controls (Figure 2).

### Example 5: Induction of fetal hemoglobin in CD34⁺ bone marrow cell cultures

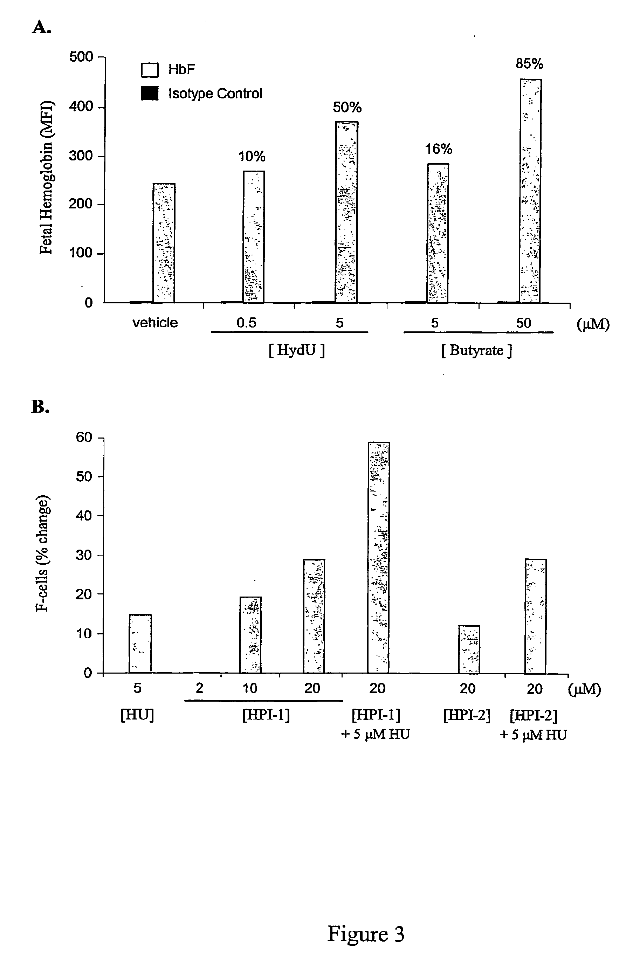

Human bone marrow progenitor cells represent a physiological source of F-cells and HbF production in anemic patients. CD34⁺ primary progenitor cells (Cambrex) from healthy donors were cultured in a 2-phase culture protocol as described in Example 2. The assay conditions in CD34⁺ cells were established using HU and butyrate as positive inducers of HbF. As can be seen in Figure 3A, HU and butyrate, added during the EPO-dependent Phase II expansion culture, stimulated increases of 50% and 85%, respectively, in HbF synthesis compared to vehicle controls.

HPIs were then assayed for their ability to stimulate an increase in F-cells and HbF synthesis, either alone or in combination with HU, in CD34⁺ progenitor cell cultures. Cells were treated with HPI-1, HPI-2, HU, or a combination thereof only during Phase II culture. As can be seen in Figure 3B, HPI-1 alone stimulated a 29% increase in F-cells over untreated cultures, and HPI-1 in combination with 5 µM HU produced a 58% increase. In a similar fashion, the percentage of F-cells was increased by 12% and 29% by HPI-2 alone and in combination with 5 µM HU, respectively (Figure 3B). Concurrent with an increase in F-cells, HbF expression was increased 36% by HPI-1 and 5% by HPI-2 in these cultures as assessed by MFI. Further, the combination of HU and HPI increased HbF synthesis above the level seen with any of the reagents alone.

Due to its cytostatic activity, hydroxyurea was normally added only to the EPO-dependent phase II expansion cultures. However, since HPIs are not cytostatic at effective concentrations, addition of HPIs to both Phases I and II was tested. When added to both phases, HPI-1 at 10 µM and 20 µM stimulated HbF expression by 12% and 47%, respectively, over untreated controls. Similarly, when HPI-2 was added during both phases, a 40% increase over controls was seen. As before, the combination of HPI, present during phase I and phase II, and HU, added only during phase II, resulted in an additive increase in HbF expression over addition of either reagent alone.

### Example 6: HPIs selectively increase F-cells and not total hemoglobin

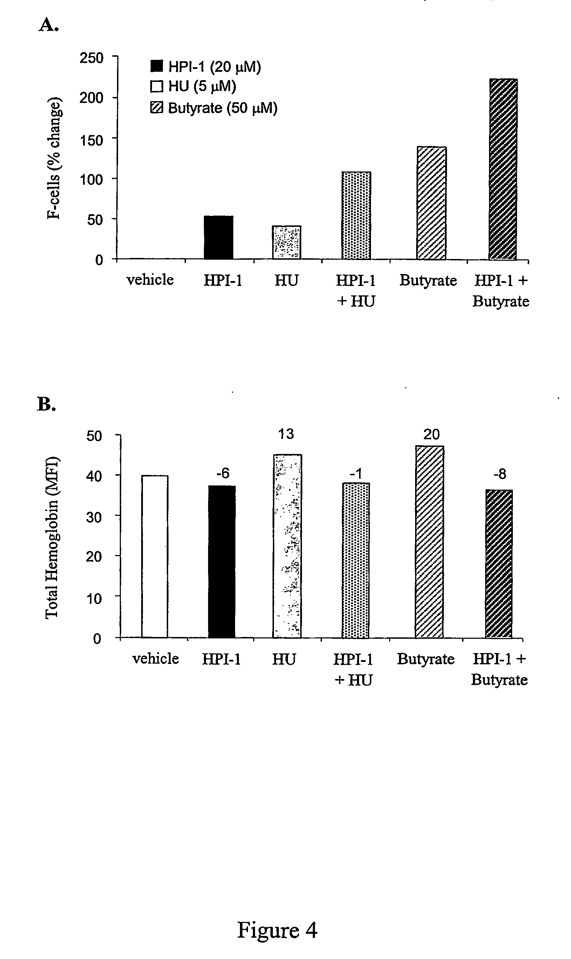

Treating CD34⁺ progenitor cells with 20 µM HPI-1 stimulated a 50% increase in F-cells, but did not cause a general increase in total hemoglobin in the cultures, indicating a specific effect on the production of fetal hemoglobin (Figures 4A and 4B). HU and butyrate alone caused increases in both F-cells and total hemoglobin, and co-treatment with HPI-1 and HU or butyrate produced an additive increase in F-cells with no significant change in total hemoglobin. (Figure 4A and 4B.)

### Example 7: HPIs increase F-cells and induce HbF composed primarily of α_{2γ2} tetramers

Intra-assay variation was examined using optimal HPI-1 concentration in triplicate. In addition, a fourth replicate was used to obtain an independent measure of HbF protein by lysing cells and assessing the absolute percentage of Hb tetramers containing HbF by ionexchange chromatography.

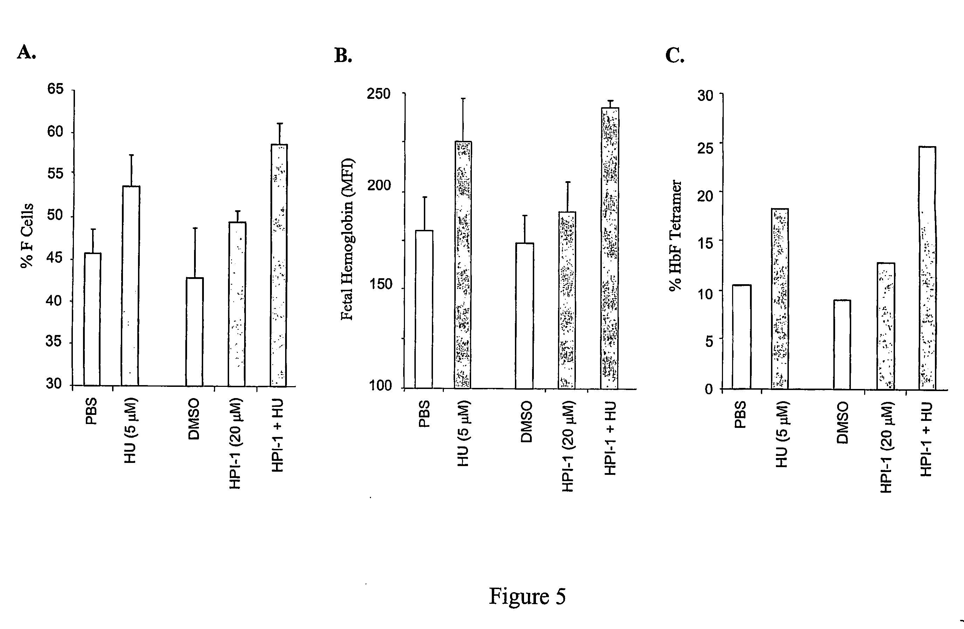

As can be seen in Figures 5A and 5B, 20 µM HPI-1 increased HbF expression as compared to vehicle control (DMSO) and was additive to the effects of HU when used together, as measured by either the percent F-cells in the cultures or the MFI for HbF, respectively. This analysis confirmed by ion exchange chromatography the increase in HbF seen previously using flow cytometry. Further, as can be seen in Figure 5C, HPIs specifically increases the percent of HbF tetramers as a function of total Hb tetramers, and the effects are additive with those of HU. Analysis of chromatograms shows only tetramers with either α₂β₂ (HbA) or α₂γ₂ (HbF), with no detectable mixed α₂β_{γ} tetramers (data not shown).

### Example 8: Representative HPIs exhibit different potencies for HbF induction

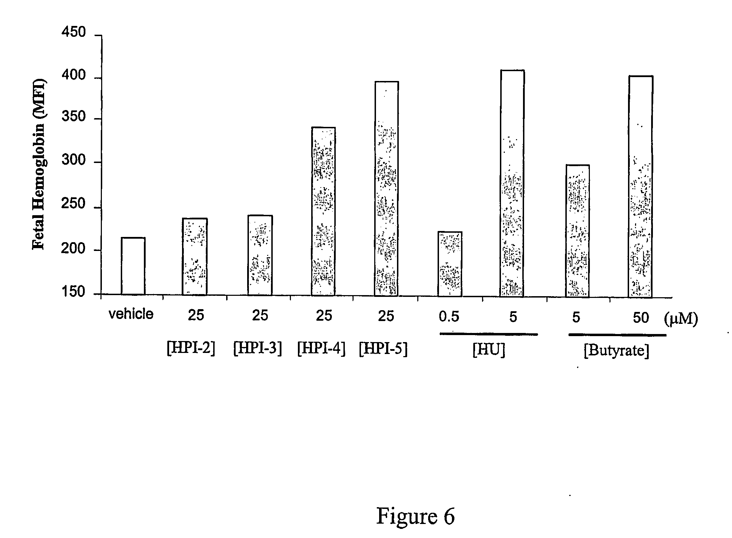

Four HPIs were tested for induction of HbF in a single assay to compare the relative potencies of the compounds. HPI-2 and HPI-3 each increased HbF expression by about 10%, HPI-4 by 56%, and HPI-5 by 75%, demonstrating a wide range of potencies for the compounds (Figure 6).

Thus, HPIs enhance HbF expression in two distinct cell populations as determined by three independent methods of HbF quantitation, and HPIs display additive effects with HU.

Various modifications of the invention, in addition to those shown and described herein, will become apparent to those skilled in the art from the foregoing description.

## Claims

1. An *ex vivo* method for increasing endogenous gamma globin (γ-globin) in a subject cell, the method comprising administering to the cell *ex vivo* an agent which increases the stability or activity of an alpha subunit of hypoxia inducible factor (HIFα).

2. An agent which increases the stability or activity of an alpha subunit of hypoxia inducible factor (HIFα) for use in treating or pretreating a subject having a disorder selected from the group consisting of β-thalassemias and sickle cell syndromes by increasing the level of fetal hemoglobin.

3. The method of claim 1 or the use of claim 2, wherein the agent increases stability or activity of HIFα by inhibiting hydroxylation of HIFα.

4. The method or use according to any one of the preceding claims, wherein HIFα is selected from the group consisting of HIF-la, HIF-2α, HIF-3α.

5. The method or agent according to any one of the preceding claims, wherein HIFα is endogenous to the subject.

6. The method or agent according to any one of the preceding claims, wherein the agent increases expression of the gene encoding γ-globin by inhibiting 2-oxoglutarate dioxygenase enzyme activity.

7. The method or agent of claim 6, wherein the 2-oxoglutarate dioxygenase enzyme is a HIF prolyl hydroxylase enzyme.

8. The method or agent of claim 7, wherein the HIF prolyl hydroxylase enzyme is selected from the group consisting of EGLN1, EGLN2, EGLN3, FIH-1.

9. The method according to any one of claims 1 and 3-8, wherein the method is for increasing the proportion of fetal hemoglobin relative to non-fetal hemoglobin produced by the cell.

10. The agent according to any one of claims 2-8, wherein the subject is infected with or at risk for being infected with a species of Plasmodium.

11. The agent according to any one of claims 2-8 or 10, wherein the agent is to be administered in combination with a second therapeutic agent that increases γ-globin production.

12. The agent of claim 11, wherein the second therapeutic agent is selected from the group consisting of hydroxyurea and 5-azacytidine.

13. The method according to any one of claims 1, 3-8 and 9, wherein the cell is derived from bone marrow.

14. The method according to any one of claims 1, 3-8 and 9, wherein the cell is selected from the group consisting of hematopoietic stem cells and blast-forming unit erythroid (BFU-E) cells.

15. A population of cells obtained by the method according to any one of claims 1, 3-8, 9 and 13-14 for use in treating or pretreating a subject having a disorder selected from the group consisting of β-thalassemias and sickle cell syndromes by increasing the level of fetal hemoglobin, wherein the cells are to be transfused into the subject.

16. The cells of claim 15, wherein the subject is infected with a species of Plasmodium.

17. The agent or cells of claims 10 or 16, wherein the species of Plasmodium is *Plasmodium falciparum*.

18. The agent or cells according to any one of claims 2-8, 10-12 or 15-17 wherein the subject is a primate.

19. The agent or cells according to any one of claims 2-8, 10-12 or 15-17 wherein the subject is a human.

20. The agent or cells according to any one of claims 2-8, 10-12 or 15-19, wherein the β-thalassemia is selected from β⁰-and β⁺-thalassemia.

21. The agent or cells according to any one of claims 2-8, 10-12 or 15-19, wherein the sickle cell syndrome is selected from sickle trait, sickle β-thalassemia, and sickle cell anemia.

22. The agent or cells according to any one of the preceding claims wherein the agent which increases the stability or activity of HIFα is a HIF prolyl hydroxylase inhibitor selected from the group consisting of N-((1-chloro-4-hydroxy-isoquinoline-3-carbonyl)-amino)-acetic acid, [(7-Bromo-4-hydroxy-isoquinoline-3-carbonyl)-amino]-acetic acid, [(1-Chloro-4-hydroxy-7-methoxy-isoquinoline-3-carbonyl)-amino]-acetic acid, [(7-Chloro-3-hydroxy-quinoline-2-carbonyl)-amino]-acetic acid, [(3-Hydroxy-6-isopropoxy-quinoline-2-carbonyl)-amino]-acetic acid, and [(4-Hydroxy-7-phenylsulfanyl-isoquinoline-3-carbonyl)-amino]-acetic acid.

23. Use of an agent which increases the stability or activity of an alpha subunit of hypoxia inducible factor (HIFα) in the manufacture of a medicament for increasing the level of fetal hemoglobin in a subject having a disorder selected from the group consisting of β-thalassemias and sickle cell syndromes.

## Patentansprüche

1. Ex-vivo-Verfahren zum Erhöhen von endogenem gamma-Globin (γ-Globin) in einer Subjektzelle, wobei das Verfahren das Verabreichen eines Mittels, das die Stabilität oder Aktivität einer alpha-Untereinheit des Hypoxie-induzierbaren Faktors (HIFα) erhöht, *ex vivo* an die Zelle umfasst.

2. Mittel, das die Stabilität oder Aktivität einer alpha-Untereinheit des Hypoxie-induzierbaren Faktors (HIFα) erhöht, für die Verwendung bei der Behandlung oder Vorbehandlung eines Subjekts, das eine Störung aufweist, ausgewählt aus der Gruppe bestehend aus β-Thalassämien und Sichelzellsyndromen, durch Erhöhen des Spiegels von fötalem Hämoglobin.

3. Verfahren gemäß Anspruch 1 oder Verwendung gemäß Anspruch 2, wobei das Mittel die Stabilität oder Aktivität von HIFα durch Hemmen der Hydroxylierung von HIFα erhöht.

4. Verfahren oder Verwendung gemäß einem der vorstehenden Ansprüche, wobei HIFα ausgewählt ist aus der Gruppe bestehend aus HIF-1α, HIF-2α, HIF-3α.

5. Verfahren oder Mittel gemäß einem der vorstehenden Ansprüche, wobei HIFα dem Subjekt endogen ist.

6. Verfahren oder Mittel gemäß einem der vorstehenden Ansprüche, wobei das Mittel die Expression des Gens, das γ-Globin codiert, durch Hemmen der 2-Oxoglutaratdioxygenase-Enzymaktivität erhöht.

7. Verfahren oder Mittel gemäß Anspruch 6, wobei das 2-Oxoglutaratdioxygenase-Enzym ein HIF-Prolylhydroxylase-Enzym ist.

8. Verfahren oder Mittel gemäß Anspruch 7, wobei das HIF-Prolylhydroxylase-Enzym ausgewählt ist aus der Gruppe bestehend aus EGLN1, EGLN2, EGLN3, FIH-1.

9. Verfahren gemäß einem der Ansprüche 1 und 3-8, wobei das Verfahren zum Erhöhen des Anteils von fötalem Hämoglobin relativ zu nicht-fötalem Hämoglobin, das von der Zelle produziert wird, ist.

10. Mittel gemäß einem der Ansprüche 2-8, wobei das Subjekt mit einer Spezies von Plasmodium infiziert ist oder unter dem Risiko steht, damit infiziert zu sein.

11. Mittel gemäß einem der Ansprüche 2-8 oder 10, wobei das Mittel in Kombination mit einem zweiten therapeutischen Mittel, das die γ-Globin-Produktion erhöht, zu verabreichen ist.

12. Mittel gemäß Anspruch 11, wobei das zweite therapeutische Mittel ausgewählt ist aus der Gruppe bestehend aus Hydroxyharnstoff und 5-Azacytidin.

13. Verfahren gemäß einem der Ansprüche 1, 3-8 und 9, wobei die Zelle aus Knochenmark stammt.

14. Verfahren gemäß einem der Ansprüche 1, 3-8 und 9, wobei die Zelle ausgewählt ist aus der Gruppe bestehend aus hämatopoetischen Stammzellen und Blast-Forming-Unit-Erythroid(BFU-E)-Zellen.

15. Population von Zellen, erhalten durch das Verfahren gemäß einem der Ansprüche 1, 3-8, 9 und 13-14 für die Verwendung bei der Behandlung oder Vorbehandlung eines Subjekts, das eine Störung aufweist, ausgewählt aus der Gruppe bestehend aus β-Thalassämien und Sichelzellsyndromen, durch Erhöhen des Spiegels von fötalem Hämoglobin, wobei die Zellen in das Subjekt zu übertragen sind.

16. Zellen gemäß Anspruch 15, wobei das Subjekt mit einer Spezies von Plasmodium infiziert ist.

17. Mittel oder Zellen gemäß Ansprüchen 10 oder 16, wobei die Spezies von Plasmodium *Plasmodium falciparum* ist.

18. Mittel oder Zellen gemäß einem der Ansprüche 2-8, 10-12 oder 15-17, wobei das Subjekt ein Primat ist.

19. Mittel oder Zellen gemäß einem der Ansprüche 2-8, 10-12 oder 15-17, wobei das Subjekt ein Mensch ist.

20. Mittel oder Zellen gemäß einem der Ansprüche 2-8, 10-12 oder 15-19, wobei die β-Thalassämie ausgewählt ist aus β⁰- und β⁺-Thalassämie.

21. Mittel oder Zellen gemäß einem der Ansprüche 2-8, 10-12 oder 15-19, wobei das Sichelzellsyndrom ausgewählt ist aus Sichelmerkmal, Sichel-β-Thalassämie und Sichelzellanämie.

22. Mittel oder Zellen gemäß einem der vorstehenden Ansprüche, wobei das Mittel, das die Stabilität oder Aktivität von HIFα erhöht, ein HIF-Prolylhydroxylasehemmer ist, ausgewählt aus der Gruppe bestehend aus N-((1-Chlor-4-hydroxyisochinolin-3-carbonyl)amino)essigsäure, [(7-Brom-4-hydroxyisochinolin-3-carbonyl)amino]essigsäure, [(1-Chlor-4-hydroxy-7-methoxyisochinolin-3-carbonyl)amino]essigsäure, [(7-Chlor-3-hydroxychinolin-2-carbonyl)amino]essigsäure, [(3-Hydroxy-6-isopropoxychinolin-2-carbonyl)amino]essigsäure und [(4-Hydroxy-7-phenylsulfanylisochinolin-3-carbonyl)amino]essigsäure.

23. Verwendung eines Mittels, das die Stabilität oder Aktivität einer alpha-Untereinheit des Hypoxie-induzierbaren Faktors (HIFα) erhöht, bei der Herstellung eines Medikaments zum Erhöhen des Spiegels von fötalem Hämoglobin in einem Subjekt, das eine Störung aufweist, ausgewählt aus der Gruppe bestehend aus β-Thalassämien und Sichelzellsyndromen.

## Revendications

1. Procédé *ex vivo* pour augmenter la gamma-globine (y-globine) endogène dans une cellule d'un sujet, le procédé comprenant l'administration à la cellule ex vivo d'un agent qui augmente la stabilité ou l'activité d'une sous-unité alpha du facteur inductible par l'hypoxie (HIFα).

2. Agent qui augmente la stabilité ou l'activité d'une sous-unité alpha du facteur inductible par l'hypoxie (HIFα) pour utilisation dans le traitement ou le prétraitement d'un sujet ayant un trouble choisi dans le groupe consistant en les β-thalassémies et les drépanocytoses, par augmentation du niveau d'hémoglobine foetale.

3. Procédé de la revendication 1 ou utilisation de la revendication 2, où l'agent augmente la stabilité ou l'activité du HIFα par inhibition de l'hydroxylation du HIFα.

4. Procédé ou utilisation selon l'une quelconque des revendications précédentes, où le HIFα est choisi dans le groupe consistant en le HIF-1α, le HIF-2α, le HIF-3α.

5. Procédé ou agent selon l'une quelconque des revendications précédentes, dans lequel le HIFα est endogène au sujet.

6. Procédé ou agent selon l'une quelconque des revendications précédentes, dans lequel l'agent augmente l'expression du gène codant pour la γ-globine par inhibition de l'activité de l'enzyme 2-oxoglutarate dioxygénase.

7. Procédé ou agent de la revendication 6, dans lequel l'enzyme 2-oxoglutarate dioxygénase est une enzyme HIF prolyl-hydroxylase.

8. Procédé ou agent de la revendication 7, dans lequel l'enzyme HIF prolyl-hydroxylase est choisie dans le groupe consistant en EGLN1, EGLN2, EGLN3, FIH-1.

9. Procédé selon l'une quelconque des revendications 1 et 3-8, le procédé consistant à augmenter la proportion d'hémoglobine foetale par rapport à l'hémoglobine non foetale produite par la cellule.

10. Agent selon l'une quelconque des revendications 2-8, le sujet étant infecté ou présentant un risque d'infection par une espèce de Plasmodium.

11. Agent selon l'une quelconque des revendications 2-8 ou 10, l'agent devant être administré en combinaison avec un deuxième agent thérapeutique, qui augmente la production de γ-globine.

12. Agent de la revendication 11, le deuxième agent thérapeutique étant choisi dans le groupe consistant en l'hydroxyurée et la 5-azacytidine.

13. Procédé selon l'une quelconque des revendications 1, 3-8 et 9, dans lequel la cellule dérive de la moelle osseuse.

14. Procédé selon l'une quelconque des revendications 1, 3-8 et 9, dans lequel la cellule est choisie dans le groupe consistant en les cellules souches hématopoïétiques et les cellules érythroïdes progénitrices (BFU-E).

15. Population de cellules obtenues par le procédé selon l'une quelconque des revendications 1, 3-8, 9 et 13-14, pour utilisation dans le traitement ou le prétraitement d'un sujet présentant un trouble choisi dans le groupe consistant en les β-thalassémies et les drépanocytoses, par augmentation du niveau d'hémoglobine foetale, les cellules devant être transfusées au sujet.

16. Cellules de la revendication 15, le sujet étant infecté par une espèce de Plasmodium.

17. Agent ou cellules des revendications 10 ou 16, l'espèce de Plasmodium étant *Plasmodium falciparum*.

18. Agent ou cellules selon l'une quelconque des revendications 2-8, 10-12 ou 15-17, le sujet étant un primate.

19. Agent ou cellules selon l'une quelconque des revendications 2-8, 10-12 ou 15-17, le sujet étant un humain.

20. Agent ou cellules selon l'une quelconque des revendications 2-8, 10-12 ou 15-19, la β-thalassémie étant choisie parmi la β⁰- et la β⁺-thalassémie.

21. Agent ou cellules selon l'une quelconque des revendications 2-8, 10-12 ou 15-19, la drépanocytose étant choisie parmi le trait thalassémique, la β-thalassodrépanocytose, et l'anémie à hématies falciformes.

22. Agent ou cellules selon l'une quelconque des revendications précédentes, l'agent qui augmente la stabilité ou l'activité du HIFα étant un inhibiteur de la HIF prolyl-hydroxylase choisi dans le groupe consistant en l'acide N-((1-chloro-4-hydroxyisoquinoléine-3-carbonyl)-amino)-acétique, l'acide [(7-bromo-4-hydroxy-isoquinoléine-3-carbonyl)-amino]-acétique, l'acide [(1-chloro-4-hydroxy-7-méthoxyisoquinoléine-3-carbonyl)-amino]-acétique, l'acide [(7-chloro-3-hydroxy-quinoléine-2-carbonyl)-amino]-acétique, l'acide [(3-hydroxy-6-isopropoxy-quinoléine-2-carbonyl)-amino]-acétique et l'acide [(4-hydroxy-7-phénylsulfanyl-isoquinoléine-3-carbonyl)-amino]-acétique.

23. Utilisation d'un agent qui augmente la stabilité ou l'activité d'une sous-unité alpha du facteur inductible par l'hypoxie (HIFα) pour la fabrication d'un médicament destiné à augmenter le niveau d'hémoglobine foetale chez un sujet présentant un trouble choisi dans le groupe consistant en les β-thalassémies et les drépanocytoses.
